# EUROPEAN PATENT APPLICATION

(11) **EP 1 164 194 A2**
(43) Date of publication of application: **19.12.2001**
(21) Application number: 00311768.6
(22) Date of filing: 29.12.2000
(51) Int. Cl.: C12N 15/82, A01H 5/00, A01H 5/10

(54) **Protein production in transgenic plant seeds**

(30) Priority: 12.06.2000 US 592427
(71) Applicant: Academia Sinica, Nan-Kang, Taipei, 115 (TW)
(72) Inventor: YU, Su-May, Nankang Taipei 115 (TW); CHENG, Kuo-Joan, Richmond B.C. VGY 3W8 (CA); LIU, Li-Fei, Taipei 100 (TW); SHAW, Jei-Fu, Nankang Taipei 115 (TW)
(74) Representative: Sexton, Jane Helen

(57) **Abstract**

A transgenic plant, the genomic DNA of which comprises a gene, the said gene comprising a promoter operably linked to a nucleotide sequence encoding a hydrolytic enzyme, wherein the promoter drives expression of the hydrolytic enzyme in a developing seed, a germinating seed, a germinated seed, or a seedling of the transgenic plant.

### Annex to the application documents - subsequently filed sequences listing

SEQUENCE LISTING

<110>  Academia Sinica

<120>  Protein production in transgenic plant seeds

<130>  N81353 JHS

<140>  00311768.6

<141>  2000-12-29

<150>  US 09/592,427

<151>  2000-06-12

<160>  28

<170>  PatentIn version 3.0

<210>  1

<211>  31

<212>  DNA

<213>  artificial

<220>

<223>  PCR primer

<400>  1
ttaagcgata tcgccaaggc cccggaacag a                                    31

<210>  2

<211>  24

<212>  DNA

<213>  artificial

<220>

<223>  sequence

<400>  2
acgcaggatc cacctcataa aacc                                              24

<210>  3

<211>  31

<212>  DNA

<213>  artificial

<220>

<223>  PCR primer

<400>  3
acggcggata tccagagtga gccggagctg a                                      31

<210>  4

<211>  31

<212>  DNA

<213>  artificial

<220>

<223>  PCR primer

<400>  4

aggttggatc cttacaaact gcacgaaggg t 31

<210> 5

<211> 27

<212> DNA

<213> artificial

<220>

<223> PCR primer

<400> 5
cgggatccta gctttagcta tagcgat 27

<210> 6

<211> 27

<212> DNA

<213> artificial

<220>

<223> PCR primer

<400> 6
tccccgcggg tcctctaagt gaaccgt 27

<210> 7

<211> 27

<212> DNA

<213> artificial

<220>

<223>  PCR primer

<400>  7
tccggatccc agatcgttca aacattt                                          27

<210>  8

<211>  27

<212>  DNA

<213>  artificial

<220>

<223>  PCR primer

<400>  8
agcccgcggg atcgatctag taacat                                           26

<210>  9

<211>  29

<212>  DNA

<213>  artificial

<220>

<223>  PCR primer

<400>  9
accgggtcga cgtatacatg tcacctaca                                        29

<210>  10

<211>  27

<212>  DNA

<213>  artificial

<220>

<223>  PCR primer

<400>  10
ggtgatatcc aggacttgcc cggctgt                                          27

<210>  11

<211>  25

<212>  DNA

<213>  artificial

<220>

<223>  PCR primer

<400>  11
gctctagaaa tctgagcgca cgatg                                            25

<210>  12

<211>  27

<212>  DNA

<213>  artificial

<220>

<223>  PCR primer

<400>  12
tccccgcggt aagcattaag cagtgca                                          27

<210>  13

<211>  29

<212>  DNA

<213>  artificial

<220>

<223>  PCR primer

<400>  13
ggggaattcg atctcgattt ttgaggaat                                          29

<210>  14

<211>  29

<212>  DNA

<213>  artificial

<220>

<223>  PCR primer

<400>  14
gggggatccc atagctattt gtacttgct                                          29

<210>  15

<211>  29

<212>  DNA

<213>  artificial

<220>

<223>  PCR primer

<400>  15
gggggatccg ggattaaata gctgggcca                                          29

<210>  16

<211>  29

<212>  DNA

<213>  artificial

<220>

<223>  PCR primer

<400>  16
gggggatccc ctcactttcc gaagtggtt                                          29

<210>  17

<211>  25

<212>  DNA

<213>  artificial

<220>

<223>  PCR primer

<400>  17
cgggatcctt aagcttgcat cttga                                              25

<210>  18

<211>  33

<212>  DNA

<213>  artificial

<220>

<223>  PCR primer

<400>  18
ccggcggccg cctacatatt ttccccttgg cca                                    33


<210>  19

<211>  27

<212>  DNA

<213>  artificial


<220>

<223>  PCR primer

<400>  19
tccgagctcc agatcgttca aacattt                                           27


<210>  20

<211>  26

<212>  DNA

<213>  artificial sequence


<400>  20
agcgagctcg atcgatctag taacat                                            26


<210>  21

<211>  27

<212>  DNA

<213>  artificial

<220>

<223>  PCR primer

<400>  21
cgccgcggta gctttagcta tagcgat                                          27

<210>  22

<211>  25

<212>  DNA

<213>  artificial

<220>

<223>  PCR primer

<400>  22
tccccgcggg tcctaagtga accgt                                            25

<210>  23

<211>  28

<212>  DNA

<213>  artificial

<220>

<223>  PCR primer

<400>  23
cgcatatgtt aagcttgcat cttgattc                                         28

<210>  24

<211>  31

<212>  DNA

<213>  artificial

<220>

<223>  PCR primer

<400>  24
ccgctcgagc tacatatttt ccccttggcc a                                    31

<210>  25

<211>  28

<212>  DNA

<213>  artificial

<220>

<223>  PCR primer

<400>  25
cggaattcgc caaggcgccg gagcagac                                        28

<210>  26

<211>  28

<212>  DNA

<213>  artificial

<220>

<223>  PCR primer

<400>  26
gctctagata cgccttcgcc ggatggct                                        28

<210> 27

<211> 24

<212> DNA

<213> artificial

<220>

<223> PCR primer

<400> 27
cccgaattca tgaaatactg gcag 24

<210> 28

<211> 24

<212> DNA

<213> artificial

<220>

<223> PCR primer

<400> 28
cccgagctct tacgccttcg ccgg 24

## Description

The use of transgenic plants for the production of pharmaceutical proteins and industrial enzymes has been proposed. In general, expression of recombinant proteins relies on stable integration of a heterologous gene into a host plant genome using, e.g., *Agrobacterium-*mediated transformation or particle bombardment. In terms of cost, production of commercially valuable proteins using crops in the field is more competitive than other biological production systems (such as yeast, bacteria, or mammalian cell cultures) which require complex and high-maintenance bioreactors. Further, protein production in plant can be easily scaled-up to produce large quantities.

The invention is based on the discovery that hydrolytic enzymes have a specific activity higher than expected (as compared to, e.g., *E. coli* produced enzyme) when expressed in transgenic seeds using a transgene having a promoter active in a plant seed tissue and driving transcription of a sequence encoding the hydrolytic enzyme.

More specifically, the invention features a transgenic plant (e.g., a transgenic rice, barley, rye, or canola plant) whose genomic DNA includes a gene having a promoter (e.g., an α-amylase promoter, such as that of the *αAmy8* gene, or a glutelin promoter) and a nucleotide sequence encoding a hydrolytic enzyme and operably linked to the promoter. The promoter drives expression of the hydrolytic enzyme in a plant seed tissue of a developing seed, germinating seed, germinated seed, or seedling of the transgenic plant. The enzyme optionally hydrolyzes a substrate naturally occurring in a plant seed tissue. If the enzyme is a phytase, the enzyme can also be an acid phosphatase, such as the enzyme encoded by an *appA* gene, or a phytase from *Selenomonas ruminantium* (e.g., strain JY35, available as ATCC 55785), such as the enzyme encoded by the *phyA* gene. If the enzyme is a amylopullulanase (e.g., of *Thermoanaerobacter ethanolicus*), the enzyme can also be an α-amylase. The invention also includes a transgenic seed produced from a transgenic plant as described above. As used herein, a "seed" can be a developing seed, a mature seed, a germinating seed, a germinated seed, or a seedling. Thus, the invention further features a method of producing a hydrolytic enzyme by providing a transgenic plant of the invention, harvesting a transgenic seed from the transgenic plant, germinating the seed, and optionally isolating the hydrolytic enzyme from the transgenic seed.

The invention also features a method of producing a polypeptide by providing a transgenic plant whose genomic DNA contains a gene having an α-amylase promoter and a nucleotide sequence encoding a polypeptide and operably linked to the promoter; harvesting a transgenic seed from the transgenic plant; germinating the transgenic seed to produce a mature seed; and isolating the polypeptide from the mature seed (e.g., from an embryo or endosperm tissue of the mature seed). Also included in the invention is a method of producing a polypeptide by providing a transgenic plant whose genomic DNA contains a gene having a glutelin gene promoter and a nucleotide sequence encoding a polypeptide and operably linked to the promoter; harvesting a transgenic seed from the transgenic plant; germinating the transgenic seed; and isolating the polypeptide from an embryo or endosperm tissue of the seed.

As used herein, a "phytase" is an enzyme that reacts with phytate or phytin to release phosphorus or a moiety that contains phosphorus that is absorbable in the GI of a monogastric animal. Thus, a phosphatase that releases inorganic phosphorus or phosphate from phytate is also a phytase.

By one genetic element being "operably linked" to another is meant that one genetic element (either in a plus strand, minus strand, or double stranded form) is structurally configured to operate or affect another genetic element. For example, a promoter operably linked to a sequence encoding a polypeptide means that the promoter initiates transcription of a nucleic acid encoding the polypeptide.

Germination begins with water uptake by the seed (imbibition) and ends with the start of elongation by the embryonic axis, usually the radicle. Therefore, germination does not include seedling growth, which commences when germination terminates. For germination to be completed, the radicle must expand and penetrate the surrounding structures. A developing seed is one that is germinating or supporting growth of the seedling. As used herein, the term "seedling" means the juvenile plant grown from a germinating seed, as defined in de Vogel, "The Seedling," In: *Seedlings of Dicotyledons*, Centre for Agricultural Publishing and Documentation, Wageningen, Netherlands, pp 9-25, 1983

An α-amylase gene promoter and signal peptide sequence can be fused upstream of the enzyme open reading frame (ORF) or gene, which can be derived from various sources. The chimeric gene is then introduced into a plant (e.g., cereal) genome. For example, a malted transgenic cereal grain that produces high levels of phytase can be used for human consumption, animal feed, or food processing. Malting is the process by which grains are germinated under controlled condition and in contained facilities to produce a consistent product.

The advantages of using transgenic malted seeds to produce a hydrolytic enzyme as feed include: (1) the seeds contain a large quantity of nutrient and minerals in an easily available form that is easily transportable and not readily perishable; (2) the nutrients in the seeds are not chelated by, e.g., phytate; (3) in the case of malted seeds, little toxicity is expected for consumers of the feed, because the malted seeds have long been used in the brewing and food industries and are known to be harmless to humans and animals; and (4) the cost of manufacturing feed for monogastric animals is reduced because an exogenous enzyme does not need to be added to the raw material.

In addition, the transgenic plants of the invention, or organs and seeds thereof, can be used to produce hydrolytic enzymes for a variety of industrial processes. Examples of such applications are in feed additives for non-ruminants, in soy processing, in food processing, or in the production of inositol or inositol-phosphates from phytate.

Other features or advantages of the present invention will be apparent from the following detailed description, and also from the claims.

The following drawings illustrate the invention:

Fig. 1 is a line graph of pH versus phytase activity.

Fig. 2 is a line graph of pH versus acid phosphatase activity.

Fig. 3 is a bar graph of various transgenic plants versus amount of APU produced therein. The filled bars represent plant samples grown in sucrose. The open bars represent plant samples grown without sucrose. "NT" is the non-transformed control.

Figs. 4 and 5 ars bar graphs of various transgenic lines versus amount of APU. "NT" is the non-transformed control.

Fig. 6 is a bar graph of various transgenic lines versus specific activity for APU.

Figs. 7 and 8 are bar graphs of various transgenic lines versus sugar concentration. "NT" is the non-transformed control.

The invention relates to transgenic plants that produce seeds useful as the raw material for a phosphorus or carbohydrate-rich feed. The superior quality of the seeds and the resulting feed is due to the insertion of a transgene containing a promoter active in a plant seed tissue and that highly expresses an enzyme in a seed tissue of a transgenic plant. Of course, the enzyme may be expressed elsewhere in a transgenic seed or plant without affecting the advantages that the invention offers. Seeds produced from such a plant can have reduced phytate and increased dietary phosphorus content, or reduced complex carbohydrates and increased simple carbohydrates, compared to a control plant that does not contain the transgene.

Phytate (*myo*-inositol hexakisphosphate) is the main storage form of phosphorus in many seeds. Phytin is the insoluble mixed potassium, magnesium, and calcium salt of *myo*-inositol hexaphosphoric acid (phytic acid). In cereals, oil seeds, and legumes, phytate accumulates in seeds during maturation and accounts for 50 to 80% of total phosphorus content of the seed. Soybean and corn meal are major components of animal feed. They contain adequate phosphorus levels to meet animal growth requirements, so long as phosphorus from the phytate is digestible (i.e., absorbable) by the animal. Ruminants (multigastric animals) readily cleave phytate to release digestible phosphorus. However, monogastric animals (e.g., pigs, poultry, and fish) utilize phytate extremely poorly because they are deficient in gastrointestinal (GI) tract enzymes capable of hydrolyzing phytate. This deficiency necessitates supplementation of animal feed with phosphorus to meet dietary requirements. Further, phytate chelates important cations (including iron, magnesium, manganese, zinc, calcium, copper, and molybdenum) and forms phytate-cation-protein complexes, thereby lowering the bioavailability of minerals and amino acids in the feed. Thus, the invention provides a means to release phosphorus by degrading phytate in raw materials used for animal feed.

Phytase produced in a transgenic plant or seed of the invention can also be used in a process for steeping corn or sorghum kernels. The plant tissue or seed can be ground before adding to steeping com. Phytase liberated from the plant tissue or seed can act on phytin, which is present in many corn preparations. The transgenic cereal grains, if they are corn or sorghum grains, can produce phytase to degrade phytin in situ. Degradation of phytin in corn steeping increases the nutrient content and consequently the commercial value of corn steep liquor, which is used as animal feed or as a nutrient in microbial fermentation. Furthermore, the degradation of phytin can prevent problems relating to the accumulation of phytin deposits in filters, pipes, reactor vessels, etc. during concentration, transport and storage of corn steep liquor. The action of phytase can also accelerate the steeping and separation processes involved in corn wet milling.

In addition, the use of transgenic seeds containing starch-hydrolyzing enzymes (e.g., amylopullulanase) is compatible and desirable with currently used processes in food and wine industries for production of sugars using cereal grain starch. Rice provides the main source of food for more than 50% of the world population and is the most important and widely grown crop on earth. Due to the large biomass, rice seeds are an ideal sources of soluble sugars for the food and wine industries. Amylopullulanase (APU) from *Thermoanaerobacter ethanolicus 39E*, harboring both pullulanase and α-amylase activities, is capable of hydrolyzing both α-1,4 and α-1,6 bonds of polysaccharides and is heat stable with a catalytic optimum of 90°C. Generally, rice contains 6-10% protein and 70-80% starch by weight. APU can hydrolyze the starch to produce soluble sugars and at the same time increase the relative composition of insoluble rice flour. High protein rice flour has high nutritional value and is useful for the production of pudding, gruel, instant milk, baby food, etc. As shown below, a 2.9-kb DNA fragment of the APU gene that encodes amino acids 75 to 1029 of the mature APU (total of 1481 amino acids) of *T. ethanolicus* was isolated and characterized. This truncated APU maintains both α-amylase and pullulanase activities. The use of transgenic rice seeds containing the dual active APU in the production of sugars from seed starch simplifies the production process and significantly reduces production cost for rice flour.

The genetic elements necessary for constructing the transgene to be inserted into the plant is readily available. For example, α-amylase promoters are described in U.S. Patent No. 5,460,952 and in the Example below. Other suitable promoters are described in McElroy et al., Plant Mol. Biol. 15:257-268, 1990; Brusslan et al., Proc. Natl. Acad. Sci. USA 89:7791-7795, 1992; Wissenbach et al., Plant J. 4:411-422, 1993; Reynolds et al., Plant Mol. Biol. 29:885-896, 1995; Kaukinen et al., Plant Mol. Biol. 30:1115-1128, 1996; Beaudoin et al., Plant Mol. Biol. 33:835-846, 1997; Moore et al., Proc. Natl. Acad. Sci. USA 94:762-767, 1997; Odell et al., Nature 13:816-812, 1985; and U.S. Patent Application entitled "Plant Seedling and Embryo Promoter," filed May 22, 2000, naming inventors Su-May Yu and Yu-Chan Chao. Other genetic elements, such as cis-regulatory sequences are also available to the skilled artisan. See, e.g., U.S. Patent No. 5,969,127.

The expression constructs containing the transgene can be inserted into a vector, such as a plasmid, which is then introduced into plants using *Agrobacterium tumefaciens*-mediated DNA delivery or particle bombardment, which are standard methods known in the art.

The DNA sequence encoding an enzyme may be obtained from a variety of sources such as microbial, plant, or animal sources. For example, a DNA sequence encoding a phytase can be cloned from a ruminal bacterium by, e.g., screening of bacterial cDNA libraries or PCR amplification of nucleic acids using heterologous or degenerate probes.

The cloned genes described herein may be used as starting materials for the construction of "second generation" enzymes whose amino acid sequence has been altered by mutagenesis (e.g., site-directed mutagenesis), which have beneficial properties that differ from those of wild-type or recombinant enzymes. For example, the temperature or pH optimum, specific activity, or substrate affinity can be altered so as to be better suited for application in a defined process.

Several techniques are available for the introduction of the expression construct containing the enzyme-encoding DNA sequence into plants or cells from which transgenic plants can be produced. Such techniques include electroporation, microinjection, the ultrasonic method, polyethylene glycol-mediated protoplast transformation, the poly-L ornithine method, the calcium phosphate method, and particle bombardment.

In addition to these so-called direct DNA transformation methods, transformation systems involving vectors are widely available, such as viral vectors (e.g., from the cauliflower mosaic virus, CaMV) and bacterial vectors (e.g., from the genus *Agrobacterium*). After selection and/or screening, the protoplasts, cells, or plant parts (e.g., explants) that have been transformed can be regenerated into whole plants, using methods known in the art.

For transformation and regeneration of cereal crops, an embodiment of the present invention uses the principle of the binary vector system (Hoekema et al., Nature 303:179-180, 1983; and European Patent Application No. 0120516) in which *Agrobacterium* strains containing a *vir* plasmid with the virulence genes and a compatible plasmid with the gene construct to be transferred. The binary vectors as used in this example contain, between the left- and right-border sequences of the T-DNA, an *hph* gene coding for hygromycin resistance and a multiple cloning site for insertion of gene constructs.

The promoter used for directing the expression of the phytase can be an α-amylase gene promoter. The α-amylase gene promoters are active in germinating seeds (Huttly et al., EMBO J. 8:1907-1913, 1989; Skriver et al., Proc. Natl. Acad. Sci. USA 88:7266-7270, 1991; Lanahan et al. Plant Cell 4:203-211, 1992; Tanida et al., Mol. Gen. Genet. 244:127-134, 1994; Itoh et al., Plant Physiol. 107:25-31, 1995), sucrose-starved suspension cells (Chan et al., J. Biol. Chem. 269:17635-17641, 1994), and other tissues and organs (Chan et al., Plant Mol. Biol. 22:491-506, 1993) of cereal crops.

The transgene can further includes a signal peptide sequence derived from the α-amylase genes or another gene that is capable of directing the recombinant proteins toward the endoplasmic reticulum, vacuole, protein body, or extracellular space. Other regulatory sequences, such as terminator sequences and polyadenylation signals that function in plants, can be included in the transgene. An example of one such sequence is the 3' untranslated region of the nopaline synthase (Nos) gene of *Agrobacterium tumefaciens* or the 3' untranslated region of a rice α-amylase gene (U.S. Patent No. 5,969,127).

Once a transgenic plant of the invention is produced, expression in tissues other than a seed tissue can be beneficial. For example, the transgenic plant can be used as a producer of a recombinant phytase, whether the phytase is isolated from a leaf, sheath, stem, or root of the transgenic plant.

Phytase activity can be measured using any method known in the art, including ELISA, Western blotting, and direct enzyme assays using colorimetric techniques or native gel assays. See e.g., Shimizu, Biosci. Biotech. Biochem. 56:1266-1269, 1992; Yanke et al., Microbiology 144:1565-1573, 1998; and Kim et al., Enzyme Microb. Technol. 22:2-7, 1998.

The transgenic plants, plant organs, or malted seeds can be used directly, i.e., without further processing, or can first be processed via conventional means such as grinding to a consistency suitable for a particular use. Alternatively, the phytase can be extracted from the plant, plant organ, or malted seeds and, if desired, purified before use using conventional extraction methods and purification techniques. See, e.g., Laboure et al., Biochem. J. 295:413-419, 1993; and Golovan et al., Can. J. Microbiol. 46:59-71, 2000.

Without further elaboration, it is believed that one skilled in the art can, based on the above disclosure, and the production of transgenic plants and seeds as described below, utilize the present invention to its fullest extent. The following examples are to be construed as merely illustrative of how one skilled in the art can isolate and use the transgenic plants of the invention, and are not limitative of the remainder of the disclosure in any way. Any publications, patents, or patent applications cited in this disclosure are hereby incorporated by reference.

### EXAMPLE 1

The methods, materials, and procedures used in the present example are first described.

### Methods and Materials

*Plant Material.* The rice variety used in this example was *Oryza sativa* L. cv. Tainung 67. Immature seeds were dehulled, sterilized with 2.4% NaOCl for 1 hour, washed extensively with sterile water, and placed on N6D agar medium for callus induction as described in Toki, Plant Mol. biol. Rep. 15:16-21, 1997. After one month, calli derived from scutellae were subcultured in fresh N6D medium for transformation or in a liquid MS medium containing 3% sucrose and 10 µM 2,4-D to establish a suspension cell culture as previously described in Yu et al., J. Biol. Chem. 266:21131-21137, 1991.

*Plasmids.* Plasmid αAmy8-C carries a 1.4-kb rice α-amylase cDNA insert in pBluescript KS+ (Stratagene) (Yu et al., Gene 122:247-253, 1992). Plasmid pRY18 carries a 3.8 kb DNA fragment that contains a rice genomic rDNA cluster, including the 3' half portion of 18S rRNA gene, the complete 5.8S rRNA gene, and the 5' half portion of the 25S rRNA gene in pUC13 (Sano et al., Genome 33:209-218, 1990). Plasmid RAMYG6a contains the 3' half portion and 3' flanking region of αAmy8 genomic DNA and was generated from screening a rice genomic DNA library (Clontech) using αAmy8-C as a probe. The selected clone was subsequently subcloned from a positive EMBL-3 phage clone into pBluescript (Stratagene). Plasmid RAMYG17 contains the 5' flanking region, the entire coding region, and the 3' flanking region of αAmy7 genomic DNA and was generated from screening a rice genomic DNA library (Clontech) using αAmy8-C as a probe. Further details regarding RAMYG6a and PAMYG17 can be found in Ho et al., Master's Thesis, Department of Biology, National Taiwan Normal University, 1991. The selected clone was subsequently subcloned from a positive EMBL-3 phage clone into pBluescript.

*Plasmid Construction.* The 5' end of the *S. ruminantium* phytase gene (*SrPf6*) was modified by PCR using the plasmid pSrPf6 (U.S. Patent No. 5,939,303) as a DNA template and the primers 5'-TTAAGCGATATCGCCAA GGCCCCGGAACAGA-3' (SEQ ID NO:1; *Eco*RV site underlined) and 5'-ACGCAGGATCCACCTCATAAAACC-3' (SEQ ID NO:2; *Bam*HI site underlined) to yield a recombinant gene in which an *Eco*RV site was incorporated immediately 5' to the codon (bolded in. SEQ ID NO:1) specifying the first amino acid of the mature, processed phytase enzyme and a *Bam*HI site at the end of the phytase gene. The *Eco*RV site allows for the fusion of the phytase gene to the signal peptide sequence of the α-amylase gene *αAmy8*. The PCR product was subcloned into the *Eco*RV and *Bam*HI sites of pBluescript (Stratagene) to generated pBS/SrPf6.

The *E. coli* phosphatase gene *appA* was similarly modified by PCR at the 5' and 3' ends using plasmid pET-appA (Golovan et al., Can. J. Microbiol. 46:59-71, 2000) as a DNA template and the oligonucleotides 5'-ACGGCGGATATCCAGAGTGAGCCGG AGCTGA-3' (SEQ ID NO:3; *Eco*RV site underlined) and 5'-AGGTTGGATCCTTACA AACTGCACGAAGG GT-3' (SEQ ID NO:4; *Bam*HI site underlined). The resulting PCR product was subcloned into pBluescript to generate pBS/appA.

A 1.2 kb promoter and signal peptide region of *αAmy8* was excised with *Sal*I and *Hind*III from pAG8 (Chan et al., Plant Mol. Biol. 22:491-506, 1993) and subcloned into pBluescript to generated pBS/8SP. The *αAmy8* 3'untranslated region (3'UTR) was PCR-amplified using RAMYG6a as the DNA template and the oligonucleotides 5'-CG GGATCCTAGCTTTAGCTATAGCGAT-3' (SEQ ID NO:5; *Bam*HI site underlined) and 5'-TCCCCGCGGGTCCTCTAAGTGAACCGT-3' (SEQ ID NO:6; *Sac*II underlined). The nopaline synthase gene (*Nos*) 3'UTRs was PCR-amplified using pBI221 (Clontech) as the DNA template and the oligonucleotides 5'-TCCGGATCCCAGATCGTTCAAAC ATTT-3' (SEQ ID NO:7; *Bam*HI site underlined) and 5'-AGCCCGCGGGATCGATCTA GTAACAT-3' (SEQ ID NO:8; *Sac*II underlined).

The *αAmy8* and *Nos* 3'UTRs were subcloned into the *Bam*HI and *Sac*II sites in pBS/8SP to generate pBS/8SP8U and pBS/8SPNos, respectively. The *SrPf6* was excised with *Eco*RV and *Bam*HI from pBS/SrPf6 and subcloned into the same sites in pBS/8SP8U and pBS/8SPNos to generate pBS/8F8U and pBS/8FN, respectively. The *appA* gene was excised with *Eco*RV and *Bam*HI from pBS/appA and subcloned into the same sites in pBS/8SP8U and pBS/8SPNos to generate pBS/8A8U, and pBS/8AN. The correct in-frame fusion of the *αAmy8* signal peptide sequence with the *SrPf6* or *appA* coding region, and the junction regions which link the *SrPf6* or *appA* coding region with the *αAmy8* 3'UTR or *Nos* 3'UTR were all verified by DNA sequencing.

The 1.7-kb promoter and signal peptide region of *αAmy7* was PCR-amplified using RAMYG17a as a DNA template and the oligonucleotides 5'-ACCGGGTCGAC GTATACATGTCACCTACA-3' (SEQ ID NO:9; *Sal*I site underlined) and 5'-GGTGATATCCAGGACTT GCCCGGCTGT-3' (SEQ ID NO:10; *Eco*RV site underlined) to yield a PCR product having a *Sal*I site at the 5' end of the *αAmy7* promoter and an *Eco*RV site immediately 3' to the signal peptide sequence. The PCR product was subcloned into the *Sal*I and *Eco*RV sites in pBluescript to generate pBS/7SP. The *αAmy7* 3'UTR was PCR-amplified using RAMYG17 as the DNA template and the oligonucleotides 5'-GCTCTAGAAATCTGAGCGCACGATG-3' (SEQ ID NO:11; *Xba*I site underlined) 5'-TCCCCGCGGTAAGCATTAAGCAGTGCA-3'(SEQ ID NO:12; *Sac*II site underlined. The PCR product was subcloned into the *Xba*I and *Sac*II sites in pBS/7SP to generate pBS/7SP7U. The *Nos* 3'UTR was excised with *Xba*I and *Sac*II from pBS/8SPN and subcloned into the same sites in pBS/7SP to generate pBS/7SPNos. The *SrPf6* gene was excised with *Eco*RV and *Bam*HI from pBS/SrPf6 and subcloned into the same sites in pBS/7SP7U and pBS/7SPNos to generate pBS/7F7U and , pBS/7FN, respectively. The *appA* gene was excised with *Eco*RV and *Bam*HI from pBS/appA and subcloned into the same sites in pBS/7SP7U and pBS/7SPNos to generate pBS/7A7U and pBS/7AN, respectively. The correct in-frame fusion of the *αAmy7* signal peptide sequence with the *SrPf6* or *appA* coding region, and the junction regions which link the *SrPf6* or *appA* coding region with the *αAmy7* 3'UTR or *Nos* 3'UTR are all verified by DNA sequencing.

The cauliflower mosaic virus 35S RNA (CaMV35S) promoter-hygromycin B phosphotransferase (*hph*) coding sequence-tumor morphology large gene 3'UTR (*tml*) fusion gene was excised with *Eco*RI from pTRA151 (Zheng et al., Plant Physiol. 97:832-835, 1991) and subcloned into the *Eco*RI site of the binary vector pPZP200 (Hajdukiewicz et al., Plant Mol. Biol. 25:989-994, 1994) to generate pPZP/HPH. pPZP/HPH was linearized with *Sal*I and blunted-ended to served as a vector for cloning. pBS/8F8U, pBS/8FN, pBS/8A8U, pBS/8AN, pBS/7F7U, pBS/7FN, pBS/7A7U, and pBS/7AN were linearized with *Pvu*II to serve as inserts for cloning. The inserts were ligated with the vector to generate pPZP/8F8U, pPZP/8FN, pPZP/8A8U, pPZP/8AN, pPZP/7F7U, pPZP/7FN, pPZP/7A7U, and pPZP/7AN, respectively.

*Rice Transformation.* Plasmids were introduced into *Agrobacterium tumefaciens* strain EHA101 (Hood et al., J. Bacteriol. 168:1291-1301, 1986) with an electroporator (BTX) following the manufacturer's instruction. Calli induced from immature rice seeds were co-cultured with *Agrobacterium* according to the methods described by Hiei et al., Plant J. 6:271-282, 1994 and Toki et al., supra.

*Northern Blot Analysis.* Total RNA was isolated from endosperms of germinating seeds as described in Yu et al., Plant Mol. Biol. 30:1277-1289, 1996 and isolated from cultured suspension cells using a TRIZOL reagent (GIBCO BRL). RNA gel blot analysis was performed as described in Thomas, Methods Enzymol. 100:255-266, 1983. Briefly, 10 µg of total RNA was electrophoresed on a 1% agarose gel containing 10 mM sodium phosphate buffer (pH 6.5), transferred to a nylon filter, and hybridized with ³²P random primer labeled *SrPf6* DNA, *appA* DNA, *αAmy8* gene-specific DNA, or rDNA probe. The *αAmy8* gene-specific DNA was prepared as described in Sheu et al., J. Biol. Chem. 271:26998-27004, 1996. The blot was visualized using auto radiography and quantified using software accompanying the of a PhosphoImager (Molecular Dynamics).

*Western Blot Analysis.* Total proteins were extracted from endosperms of germinating seeds using an extraction buffer (50 mM Tris-HCl [pH 8.8], 1 mM EDTA, 10% glycerol, 1% Triton X-100, 10 mM β-mercaptoethanol, and 0.1% sarkosyl). Western blot analysis was performed as described by Yu et al., J. Biol. Chem. 266:21131-21137, 1991.

*Phytase Activity Assay.* Protein extract was prepared from the germinating seeds as described by Li et al., Plant Physiol. 114:1103-1111, 1997. The phytase activity was determined as described in Shimizu, Biosci. Biotech. Biochem. 56:1266-1269, 1992.

### Results

*The αAmy8 promoter confers sucrose starvation-enhanced accumulation of* appA *and* SrPf6 *mRNAs in transformed rice suspension cells.* To determine whether the *αAmy8* promoter controls sugar-dependent expression of *appA* in rice, a 1.2-kb DNA fragment containing the 5' regulatory and signal peptide sequences of *aAmy8* was fused in-frame at the 5' end of the *appA* or *SrPf6* gene. The chimeric gene was inserted into a binary vector and introduced into *Agrobacterium* for rice transformation. Many transformed cell lines were obtained and eight lines were selected for further study. The transformed calli were cultured as suspension cells, and the suspension cells were then cultured in medium with or without sucrose. Total RNA was purified and subjected to gel blot analysis using *appA* DNA, *SrPf6* DNA, *αAmy8* gene-specific DNA, or rDNA as a probe. Accumulation of *appA, SrPf6*, and *αAmy8* mRNAs were detectable in cells starved of sucrose but not in cells provided with sucrose. No *appA* or *SrPf6* mRNA was detected in the non-transformed (NT) cells.

*The αAmy8 promoter confers sucrose starvation-enhanced activity of phytase in transformed rice suspension cells.* To determine whether the transformed rice suspension cells carrying an *αAmy8-appA* or *αAmy8-SrPf6* chimeric gene synthesize and secrete phytase into the culture medium, the phytase activity in suspension cells and in culture medium were determined. The phytase activity was detectable in the transformed cells and in the culture medium. In both cases, phytase activity was significantly enhanced by sucrose starvation. The phytase activity was also detectable in the non-transformed cells under sucrose starvation, which was probably caused by the endogenous phytase gene.

*The αAmy7 and αAmy8 promoters control expression of SrPf6 in germinating transgenic rice seed.* Transgenic rice plants were regenerated from the transformed rice calli carrying *αAmy7-SrPf6* or *αAmy8-SrPf6.* The T1 seeds of some transgenic lines were randomly selected for further study. To determine the role of the *αAmy7* and *αAmy8* promoters in the expression of *appA* and *SrPf6* genes during seed germination, the transgenic rice seeds were germinated for 5 days. Total RNA was purified from the entire germinated seed (including the shoots, roots, and endosperms) and subjected to gel-blot analysis using *SrPf6* DNA, *αAmy8* gene-specific DNA, or rDNA as a probe. Under the control of *αAmy7* promoter, expression of *SrPf6* could be detected in some of the germinated seeds. Under the control of *αAmy8* promoter, expression of *SrPf6* could be detected in all the germinated seeds. No *SrPf6* mRNA was detected in non-transformants.

*The αAmy8 promoters control expression of appA in germinating transgenic rice seed.* Transgenic rice plants were regenerated from the rice calli carrying *αAmy8-appA.* The T1 seeds of some transgenic lines were randomly selected for further study. Seeds were germinated for 5 days and total RNA was purified from the entire germinated seeds (including the shoots, roots, and endosperms) and subjected to gel-blot analysis using *appA* DNA, *αAmy8* gene-specific DNA, or rDNA as a probe. Under the control of the *αAmy8* promoter, expression of *appA* could be detected in all germinated seeds. No *appA* mRNA was detected in the non-transformants.

*The αAmy8 promoter confers high phytase activity in germinating transgenic rice seeds.* To determine the expression level of phytase in germinated transgenic seeds, phytase activity in the T1 transgenic seeds were analyzed. The transgenic rice seeds were germinated for 5 days. Cell extract was prepared from the entire germinated seeds (including the shoots, roots, and endosperms) and subjected to phytase activity analysis. Phytase activity was higher in all the germinated transgenic seeds carrying the *αAmy8-SrPf6-Nos* chimeric gene than in the non-transformants. One of the transgenic lines, 8FN-8, had significantly higher phytase activity as compared with other transgenic lines. Phytase activity was higher in all the germinated transgenic seeds carrying the *αAmy8-appA* chimeric gene than in the non-transformant. One transgenic line, 8AN-14 had significantly higher phytase activity as compared with other transgenic lines including 8FN-8.

*The introduced phytase and acid phosphatase genes are inherited to the progenies of transgenic rice.* To determine whether the introduced *appA* and *SrPf6* DNAs were inherited to the progenies of T1 transgenic rice, the T2 seeds of transgenic lines 8AN-14 and 8FN-8 were germinated for 5 days. Total RNA was purified from the entire germinated seeds and subjected to gel blot analysis using *appA* DNA, *SrPf6* DNA, or rDNA as a probe. Expression of *appA* and *SrPf6* was detected in most of the germinated T2 seeds.

*The αAmy8 promoter confers temporal expression of phytase in germinating transgenic rice seed.* To determine the expression pattern of phytase in germinated transgenic rice seed carrying *αAmy8-appA* or *αAmy8-SrPf6*, the T2 seeds of transgenic lines 8FN-8-8 and 8AN-14-10 were germinated for various lengths of time, and phytase activity was determined. The phytase activity in the transgenic seeds increased as germination proceeded and reached a peak at days 5-6 while declining afterwards. Phytase activity was also detectable in the non-transformed seeds, but remained at low level throughout the entire germination period.

*The molecular weight of phytase produced in germinating transgenic rice seeds is similar to that produced in* Pichia pastoris. *SrPf6* was cloned into a plasmid under the control of the glyceraldehyde-3-phosphate dehydrogenase (GAP) promoter and overexpressed in *Pichia pastoris.* The SrPf6 cDNA was PCR-amplified using pSrPf6 as the DNA template and oligonucleotides 5'-CGGAATTCGCCAAGGCGCCGGAGC AGAC-3' (EcoRI site underlined) as 5' primer and 5'-GCTCTAGATACGCCTTC GCCGGATGGCT-3' (XbaI site underlined) as 3' primer. The DNA fragment containing SrPf6 cDNA was digested with EcoRI and XbaI and ligated into the same sites in pGAPZaA (Invitrogen) to generate pGAP-SrPf6. SrPf6 was led by a signal peptide α-factor and was under the control of GAP promoter. pGAP-SrPf6 was linearized by restriction enzyme AvrII and transferred into *P. pastoris* host strain SMD1168H by electroporation. The transformed cells were plated on YPDS (1% yeast extract, 2% peptone, 2% dextrose, 1 M sorbitol, pH 7.5) plus zeocin (100 mg/ml) at 30°C for 3 days. Production and purification of phytase were performed according to the manufacturer's instruction provided with pGAPZaA.

To compare the molecular weight of phytase produced in germinating transgenic rice seeds with that of the phytase overexpressed in *Pichia*, seeds of transgenic rice line 8FN-8 were germinated for 5 days. Total proteins were extracted from the entire germinated seeds and subjected to Western blot analysis using phytase polyclonal antibodies. The full-length cDNA of SrPf6 was PCR-amplified using plasmid pSrPf6 as DNA template and oligonucleotides 5'-CCCGAATTCATGAAATACTGGCAG-3' (EcoRI site underlined) as 5'-primer and 5'-CCCGAGCTCTTACGCCTTCGCCGG-3' (SacI site underlined) as 3' primer. The amplified DNA fragment was digested with EcoRI and SacI and ligated into the same sites in pET20b(+) (Novagen) to generate pET-SrPf6. pET-SrPf6 was transferred to *E. coli* strain BL21 (DE3) and expressed. Purification of phytase was performed according to the instruction provided by Novagen. One hundred micrograms of purified phytase was injected into a New Zealand White rabbit successively at 4-6-week interval according to the methods generally described in Williams et al., "Expression of foreign proteins in *E. coli* using plasmid vectors and purification of specific polyclonal antibodies," In: *DNA Cloning 2. Expression Systems: A Practical Approac*h, Glover et al., eds., IRL Press, New York, 1995.

The molecular weight of the full-length phytase produced by germinating transgenic seeds was similar to that produced by *Pichia.* It was known that the phytase encoded by *SrPf6* had a molecular weight of 36.5 kD when expressed in *E. coli* (U.S. Patent No. 5,939,303). The molecular weight of phytase expressed in *Pichia* was about 36.5 kD and 38 kD. Similarly, when the phytase was expressed in transgenic rice seeds the molecular weight was about 36.5 kD and 40 kD. It was known that there is one potential glycosylation site in the primary structure of SrPf6 (U.S. Patent No. 5,939,303). The 38 kD and 40 kD phytases expressed in *Pichia* and transgenic rice seeds, respectively, were probably caused by differential glycosylation. One protein with molecular weight of 34 kD was also present in the germinating rice seeds, which could have been a degradation product of the phytase. These results indicate that the mature phytase produced in the transgenic plants and seeds described herein were properly post-translationally modified.

*Transgenic germinated rice seed/seedling contained phytase with high specific activity and broad pH profiles for high activity.* Unexpectedly, it was also discovered that the phytase specific activity expressed in the germinated transgenic seeds is two fold higher than that expressed in *E. coli.* The reason for this surprising result could be that there are many endogenous hydrolytic enzymes simultaneously expressed in the germinated seeds. These hydrolytic enzymes may have a synergistic effect on the phytase activity present in germinating seeds. Alternatively, or in conjunction, the post-translationally modified phytase in the germinating seeds may have increased the enzyme's specific activity, which resulted in the observed increase in phytase activity. Thus, besides the advantages of expressing phytase in transgenic malted seeds, the specific activity of the recombinantly produced enzyme was also increased.

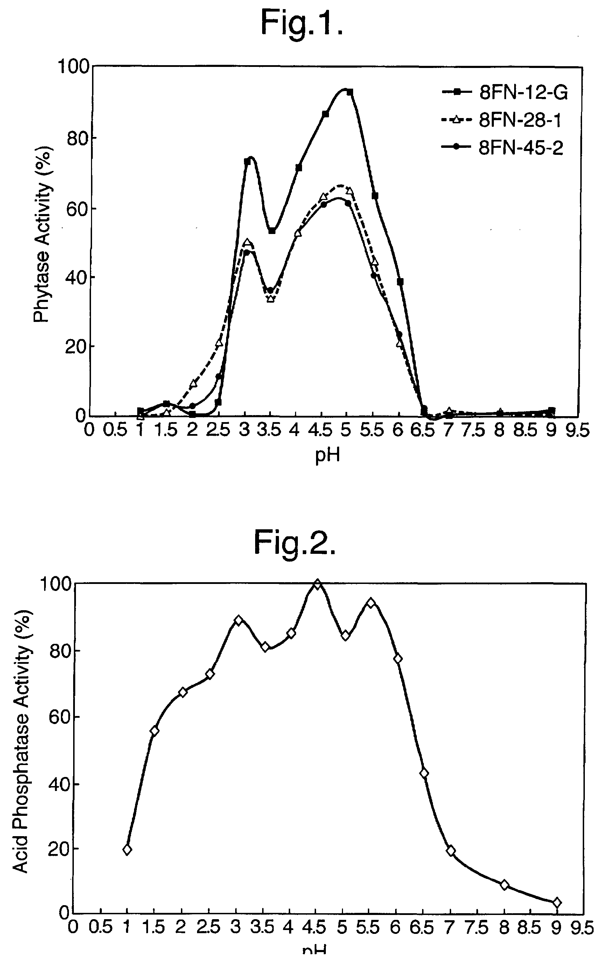

The ruminal bacterial phytase has a pH optimum of 4.0-5.5 (U.S. Patent No. 5,939,303). To determine the optimal pH of phytase expressed in the rice seeds described above, T2 seeds of three transgenic rice lines were germinated for 5 days, and the optimal pH for phytase expressed in rice germinated seed/seedling was determined. The results showed that the activity of phytase expressed in all three transgenic rice lines was optimal at pH 3 and pH 4.5-5.0 (Fig. 1). In the digestive tract of monogastric animals, the pH ranges from 2-3 in the stomach and 4-7 in the small intestine. The broader and more acidic optimal pH profile of phytase expressed in rice allows the enzyme to function well in the stomach and small intestine of animals and is therefore an advantage for its use as a feed additive. The reason for this surprising result could be that the phytase expressed in rice was post-translationally modified, which resulted in the increase in activity and/or stability over a broad range of pH.

The *E. coli* acid phosphatase has a pH optimum of 2.5 (Dassa et al., J. Biol. Chem. 257:6669-6676, 1982; and Dassa et al., J. Bacteriol. 172:5497-5500, 1990). To determine the optimal pH of acid phosphatase expressed in rice, T2 seeds of transgenic rice line 8AN-14-6 were germinated for 5 days, and the optimal pH for phytase expressed in germinated rice seed/seedling was determined. The results show that activity of acid phosphatase expressed in rice was optimal over a pH range of 3-5.5 (Fig, 2), As with the ruminal bacterial phytase expressed in rice, the broader and more acid optimal pH profile of *E. coli* acid phosphatase would allow the enzyme to function well in the stomach and small intestine of animals and is therefore an advantage for its use as a feed additive. The reason for this surprising result also could be that the acid phosphatase expressed in rice was post-translationally modified, which resulted in increase in activity and/or stability over a broad range of pH.

### EXAMPLE 2

The methods, materials, and procedures used in the present example are first described.

### Materials and Methods

*Plant Material.* All pant materials were prepared as described in Example 1.

*Preparation of genomic DNA.* Rice seeds were germinated and grown in the dark for 1 week. Bacteria *T. ethanolicus* 39E was obtained from the American Type Culture Collection (ATCC 53033). The bacterial and rice genomic DNA was purified according to the method described in Sheu et al., J. Biol. Chem 271:26998-27004, 1996.

*PCR.* The 1351-bp glutelin gene promoter region (Fragment I; Zheng et al., Plant J. 4:357-366, 1993; and Wu et al., Plant Cell Physiol. 39:885-889, 1998) was PCR-amplified using rice genomic DNA as template and primers B1-5 (5'-GGGGAATTCGA TCTCGATTTTTGAGGAAT-3' [SEQ ID NO:13], EcoRI site underlined) and B1-3 (5'-GGGGGATCCCATAGCTATTTGTACTTGCT-3' [SEQ ID NO:14], BamHI site underlined). The glutelin gene promoter plus 75-bp putative signal peptide sequence (Fragment II) was PCR-amplified using rice genomic DNA as template and primers B1-5 and B1-sp (5'GGGGGATCCGGGATTAAATAGCTGGGCCA-3' [SEQ ID NO:15], BamHI site underlined). The glutelin gene promoter plus putative signal peptide and propeptide sequences (Fragment III) was PCR-amplified using rice genomic DNA as template and primers B1-5 and B1-pro (5'GGGGGATCCCCTCACTTTCCGAAG TGGTT-3' [SEQ ID NO:16], BamHI site underlined).

The truncated *apu* ORF encoding only amino acid 75 to 1029 was PCR-amplified using the genomic DNA of *T. ethanolicus* 39E as template and oligonucleotides 5'-CGG GATCCTTAAGCTTGCATCTTGA-3' (SEQ ID NO:17; BamHI site underlined) as forward primer and 5'-CCGGCGGCCGCCTACATATTTTCCCCTTGGCGA-3' (SEQ ID NO:18; NotI site underlined) as reverse primer.

*Plasmid construction.* The PCR-amplified Fragments I, II, and III were digested with EcoRI and BamHI and subcloned into the same sites in pBluescript (Stratagene) to generate pBS-G, pBS-Gp, and pBS-Gpp, respectively. The truncated *apu* was digested with BamHI and NotI and fused downstream of the *GluB-1* promoter, *GluB-1* promoter-signal peptide sequence, and *GluB-1* promoter-signal peptide-propeptide sequence in pBS-G, pBS-Gp, and pBS-Gpp, respectively, to make translational fusions and to generate plasmids pBS-G-apu, pBS-Gp-apu, and pBS-Gpp-apu, respectively. The nopaline synthase gene (Nos) 3' untranslated (UTR) was PCR-amplified using pBI221 (Clontech) as DNA template and oligonucleotide 5'-TCCGAGCTCCAGATCGTTC AAACATTT-3' (SEQ ID NO:19; SacI site underlined) as forward primer and oligonucleotide 5'-AGCGAGCTCGATCGATCTAGTAACAT-3' (SEQ ID NO:20; SacI underlined) site as reverse primer. The Nos 3'UTR was digested with SacI and fused downstream of *apu* in pBS-G-apu, pBS-Gp-apu, and pBS-Gpp-apu to generate pBS-G-apu-Nos, pBS-Gp-apu-Nos, and pBS-Gpp-apu-Nos, respectively.

The 1.2 kb promoter and signal peptide sequence of *αAmy8* was excised with Sail and HindIII from pAG8 (Chan et al., Plant Mol. Biol. 22:491-506, 1993) and subcloned into pBluescript to generate pBS/8sp. The *αAmy8* 3'UTRs was PCR-amplified using RAMYG6a as a DNA template and oligonucleotide 5'-CGCCGCGGTAGCTTTA GCTATAGCGAT-3' (SEQ ID NO:21; SacII site underlined) as forward primer and oligonucleotide 5'-TCCCCGCGGGTCCTCTAAGTGAACCGT-3' (SEQ ID NO:22; SacII site underlined) as reverse primer. Plasmid RAMYG6a contains the 3' half portion of the coding sequence and 3' flanking region of *αAmy8* genomic DNA and was generated by screening of a rice genomic DNA library (Clontech) using *αAmy8*-C (Yu et al., Gene 122:247-253, 1992) as a probe. The *αAmy8* 3'UTRs was subcloned into the SacII sites in pBS/8sp to generate pBS/8sp8U. The truncated *apu* was cut with BamHI and NotI and subcloned into the same sites in pBS-8sp8U to generate pBS-αAmy8-sp-apu-8U.

The 1.1-kb promoter and signal peptide sequence of *αAmy3* was excised with Sail and HindIII from p3G-132II (Lu et al., J. Biol. Chem. 273:10120-10131, 1998) and subcloned into pBluescript to generate pBS-3sp. The *αAmy3* 3'UTR was excised with HindIII and SacI from pMTC37 (Chan et al., Plant J. 15:685-696, 1998) and subcloned into the same sites in pBS-3sp to generate pBS-3sp3U. The truncated *apu* was digested with BamHI and NotI and subcloned into the same sites in pBS-3sp3U to generate pBS-αAmy3-sp-apu-3U.

The correct in-frame fusion of the *GluB, αAmy3,* and *αAmy8* signal peptide or propeptide sequence with the *apu* coding region, the junction regions which link the *apu* coding region with the *αAmy3, αAmy8*, or Nos 3'UTR were all verified by DNA sequencing. The GluB-apu-Nos, GluB-sp-apu-Nos, GluB-spp-apu-Nos, αAmy3-sp-apu-αAmy3 3'UTR. and αAmy8-sp-apu-αAmy8 3'UTR chimeric genes were excised from pBS-G-apu-Nos, pBS-Gp-apu-Nos, pBS-Gpp-apu-Nos, pBS-αAmy3-sp-apu-3U, and pBS-αAmy8-sp-apu-8U with SalI, blunt-ended, and inserted into the HindIII-digested and blunt-ended binary vector pSMY1H (Ho et al., Plant Physiol. 122:57-66, 2000) to generate pSMY1/Gapu, pSMY1/Gpapu, pSMY1/Gppapu, and pSMY1/3apu and pSMY1/8apu, respectively.

*Transformation.* Transformations were carried out as described in Example 1.

*Expression of APU in E. coli and preparation of antibodies.* The truncated *apu* encoding amino acids 75 to 1029 was PCR-amplified using genomic DNA of *T. ethanolicus* 39E as template and oligonucleotides 5'-CGCATATGTTAAGCTTGC ATCTTGATTC-3' (SEQ ID NO:23; NdeI site underlined) as forward primer and 5'-CCGCTCGAGCTACATATTTTCCCCTTGGCCA-3' (SEQ ID NO:24; Xhol site underlined) as reverse primer. The amplified DNA fragment was digested with NdeI and XhoI and ligated into the same sites in pET20b(+) (Novagen) to generate pET-APU. pET-APU was transferred to *E. coli* strain BL21 (DE3), and APU was expressed. Purification of APU was performed according to the instruction provided by Novagen. One hundred micrograms of purified APU was injected into a New Zealand White rabbit successively at 4-6 week interval according to the methods described in Williams et al., supra.

*APU activity assay and enzyme-linked immunosorbent assay (ELISA).* Rice seeds or tissues were ground in liquid N₂, lysed with a buffer (90.8 mM K₂HPO₄, 9.2 mM KH₂PO₄, 10 mM EDTA, 10% glycerol, 1% Triton X-100, and 7 mM β-mercaptoethanol) and centrifuged at 15,000 X g for 10 minutes. The supernatant was then collected. APU activity was assayed as described in Mathupala et al., J. Biol. Chem. 268: 16332-16344, 1993. ELISA was performed as described in Ausubel et al., Short Protocols in Molecular Biology, 2nd ed., In: *A Compendium of Methods from Current Protocols in Molecular Biology*, John Wiley & Sons, New York, 1992. The total protein concentration was determined using a Bio-Rad protein assay kit based on the dye binding assay of Bradford (Bradford, Anal. Biochem. 72:248-254, 1976).

*Determination of sugar concentration.* Mature rice seeds were ground to powder with a mortar and pestle. Rice seedlings were ground to powder in liquid N₂ with mortar and pestle. Water was added to the powder to make a 10% (w/v) slurry. The slurry derived from mature rice seeds was heated at 90°C for 30 minutes, total soluble sugars were extracted, and the concentration of sugars was determined as described in Chen et al., Plant J. 6:625-636, 1994. The slurry derived from rice seedlings was heated at 85°C for 2 hours, total soluble sugars were extracted, and the concentrations of glucose, fructose, sucrose, maltose, and maltotriose were determined by high-performance liquid chromatography (HPLC) as described in Shaw et al., Biosci. Biotech. Biochem. 56:1071-1073, 1992.

### Results

*Isolation of the rice glutelin gene promoter, signal peptide, and propeptide sequences.* Primers based on the nucleotide sequence of a glutelin gene (*GluB-1*) (Takaiwa et al., Plant Mol. Biol. 17:875-885, 1991) were designed, and three DNA fragments containing the *GluB-1* promoter, putative signal peptide sequence, and putative propeptide sequence were synthesized using PCR. The putative 25-amino acid signal peptide cleavage site was predicted based on a statistical method (von Heijne, J. Mol. Biol. 184:99-105, 1985). The putative 36-amino acid propeptide cleavage site was predicted based on the sweet potato sporamin propeptide sequence (Matsuoka et al., Proc. Natl. Acad. Sci. USA 88:834-838, 1991). Fragment I contained the 1351 bp *GluB-1* promoter region ending right at the translation initiation codon. Fragment II contained Fragment I plus a 75 bp signal peptide sequence. Fragment III contained Fragment II plus a 108 bp putative propeptide sequence. The purpose for including the signal peptide or propeptide region was to target APU to different cellular compartments (e.g., cytoplasm, protein body, or vacuole). Effect of cellular localization on yield, stability, and activity of APU in transgenic rice seeds could then be compared. Transformed rice suspension cells expressed and secreted APU into the culture medium.

*Transformed rice suspension cells expressed and secreted APU into the culture medium.* All plasmids were delivered into rice calli by particle bombardment or *Agrobacterium*-mediated transformation systems. The transfected rice calli were grown under hygromycin selection. Genomic Southern blot analyses of the transformed cell lines revealed that, in general, the transgenic lines obtained via the *Agrobacterium*-mediated transformation contained 1 to 2 copies and that, via the particle bombardment, the transgenic plants contained multiple copies (more than 2) of the *apu* gene in the genomes.

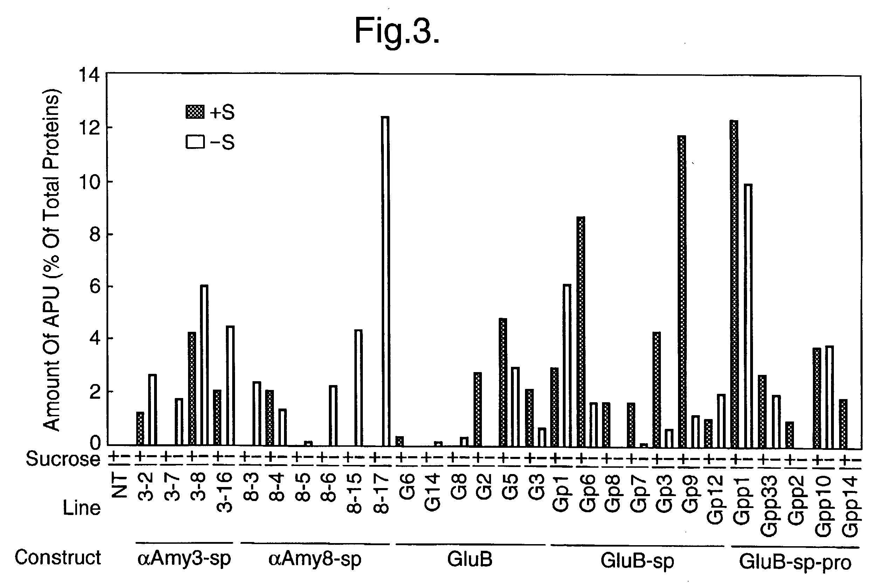

The transformed rice calli were cultured in liquid MS medium to generate suspension cell culture. Because APU was expressed with signal peptide or propeptide sequence in most of the transgenic lines, the culture media were collected for analysis of APU accumulation. The amount of APU in the media of transformed rice suspension cell cultures was determined using ELISA and the purified E. coli-expressed APU as a standard. As shown in Fig. 3, the amount of APU in media of transformed suspension cells was significantly higher than that in media of non-transformed cells. In media of cells carrying *αAmy3* or *αAmy8* promoters, the amount of APU was higher in the absence of sucrose than in the presence of sucrose.

Surprisingly, although the *GluB-1* promoter had been shown to confer endosperm-specific expression in developing rice seeds, it was found that APU was also expressed in cultured rice suspension cells. In media of cells carrying the *GluB-1* promoter, the amount of APU was higher in the presence of sucrose than in the absence of sucrose. Additionally, APU expressed without a signal peptide sequence was also present in the culture media. Computer analysis indicated that the N-terminal of APU had amino acid sequence features resembling a signal peptide sequence, which might explain the secretion of APU into the culture medium without a leader peptide added. Thus, it was discovered that APU without a signal peptide sequence could be secreted into the culture medium.

*Transgenic germinated rice seed/seedling contains high level of APU.* Transgenic calli carrying *apu* under the control of an α-amylase or glutelin gene promoter and with or without signal peptide sequence were regenerated. These transgenic plants were self-fertilized for two generations, and the T2 homozygous seeds were obtained.

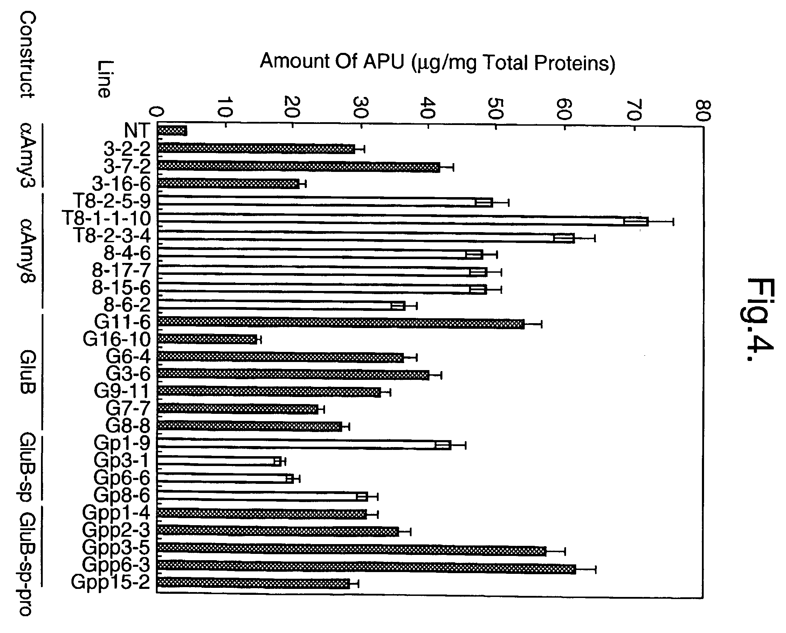

Homozygosity of transgenic seeds was determined by germination of 25 transgenic seeds in water containing 50 µg/ml hygromycin for 7 days, and then calculating the ratio between numbers of growing and non-growing seeds. Homozygous seeds was expected to germinate and grow under hygromycin treatment. Five T2 homozygous seeds from each transgenic rice line carrying various constructs were germinated and grown for 5 days. The entire germinated seeds/seedlings were collected, and the APU levels in cell extracts were determined using ELISA and purified *E. coli*-expressed APU as a standard. The average APU level expressed under the control of either an α-amylase or glutelin gene promoter was higher than that of the non-transformants (Fig. 4). The expression of APU in germinated rice seed/seedling was unexpected, because it was known that the *GluB-1* promoter was expressed specifically in endosperm during the development of the seed.

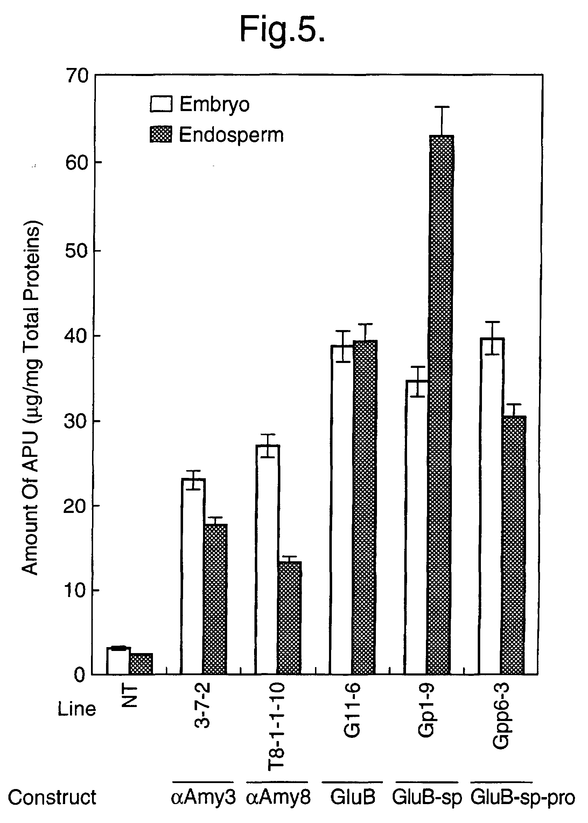

*Transgenic mature rice seeds contain high levels of APU.* Five T2 homozygous seeds of each transgenic rice line carrying a transgene were selected for APU analysis. The embryo and endosperm of mature seeds were separately collected and homogenized in buffer to form an extract. APU levels in cell extracts were determined using ELISA. The average APU level expressed under the control of either an α-amylase or glutelin gene promoter in transgenic seeds was higher than that in the non-transformed seed (Fig. 5). Unexpectedly, the *GluB-1* promoter conferred APU expression in embryo. Additionally, the fact that *αAmy3* and *αAmy8* promoters conferred APU expression in embryo and endosperm of mature seed was also unexpected because it was known that α-amylase genes were expressed specifically in endosperm or transiently in embryo of germinating seed and not in the mature seed.

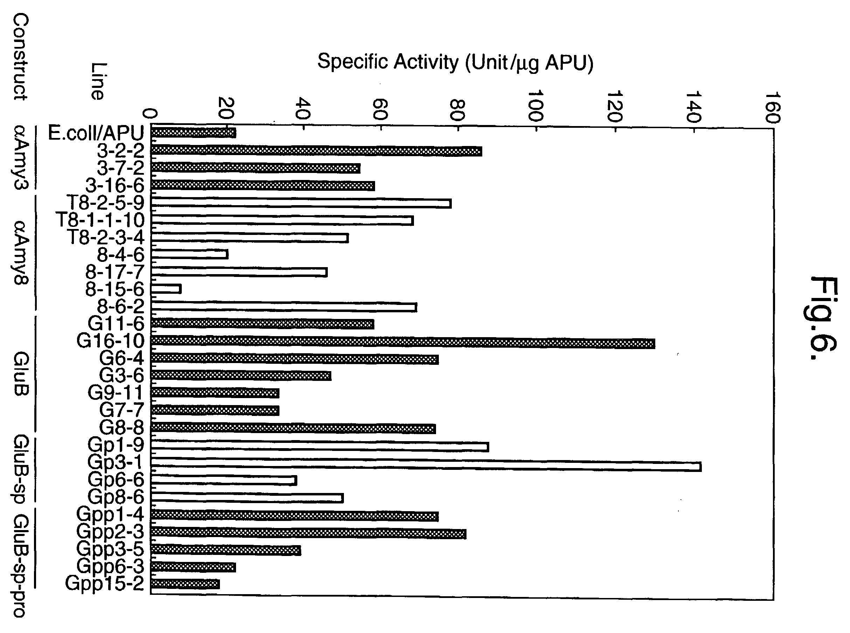

*Transgenic germinated rice seed/seedling contains APU with high specific activity.* The data in Fig. 4 indicated that the APU level was significantly higher in transgenic germinated seed/seedling than in the non-transformed control. To compare the specific activity of recombinant APU expressed in transgenic germinated seed/seedling, five T2 homozygous seeds of each transgenic rice lines carrying various constructs were germinated and grown for 5 days. Cell extracts of the entire germinated seed/seedlings were prepared, and the APU level in the cell extract was determined using ELISA. APU activity per amount of APU in the cell extracts was determined after incubation of the cell extract at 90°C for 30 minutes. As shown in Fig. 6, the specific activity of APU present in transgenic germinated seed/seedling varied from line to line regardless of the construct used. Interestingly, the specific activity of APU expressed in transgenic germinated seed/seedling was two to seven fold higher than that of the *E. coli*-expressed APU. The reason for this surprising result could be that there are many endogenous hydrolytic enzymes present in the rice endosperm. These hydrolytic enzymes may have a synergistic effect on the APU activity present in germinated seeds. Alternatively, or in conjunction, the post-translationaily modified APU in the germinated seeds may have increased the enzyme's specific activity, which resulted in the observed increase in APU activity. Note that there are three potential glycosylation sites in the APU polypeptide, which may be differentially glycosylated depending on the production method used. Thus, besides the above-mentioned advantages of expressing APU in transgenic germinated seeds, the specific activity of the recombinant enzyme was also increased.

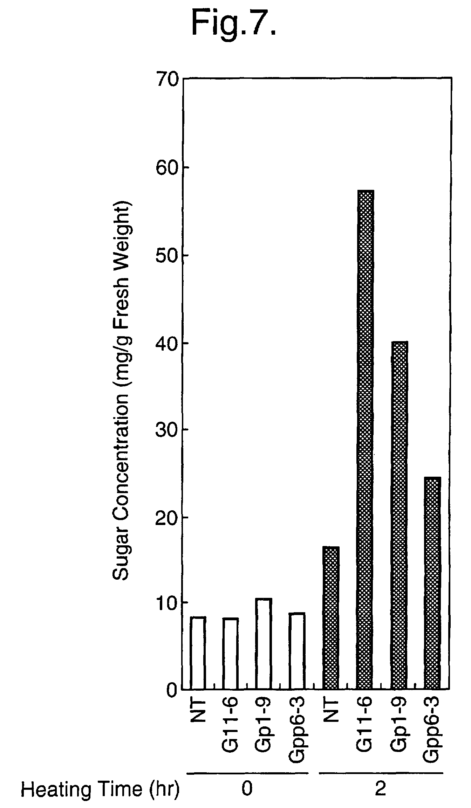

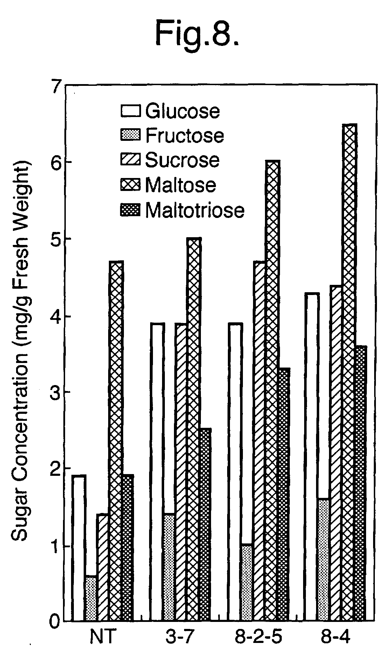

*Transgenic rice seed and germinated seed/seedling produce high level of sugars.* The results summarized in Figs. 4 and 5 revealed that APU level is significantly higher in transgenic mature seed and germinated seed/seedling, than in the non-transformed control. To determine whether transgenic seeds produce higher levels of soluble sugars than the non-transformants, five T2 homozygous seeds of each transgenic rice line carrying *apu* under the control of the *GluB-1* promoter were homogenized in water and incubated at 85°C for 2 hours. As shown in Fig. 7, the concentration of soluble sugar in transgenic seed was higher than that in the non-transformed seed. Five T2 homozygous seeds of each transgenic rice line carrying *apu* under the control of *αAmy3* and *αAmy8* promoter were also homogenized in water and incubated at 90°C for 30 minutes. As shown in Fig. 8, the concentration of sugar in germinated seed/seedling was higher than that in the non-transformed control. These results demonstrated that the increased level of APU in transgenic seed or germinated seed/seedling facilitated production of sugars from seed starch without addition of exogenous APU.

## Claims

1. A transgenic plant, the genomic DNA of which comprises a gene, the said gene comprising a promoter operably linked to a nucleotide sequence encoding a hydrolytic enzyme, wherein the promoter drives expression of the hydrolytic enzyme in a developing seed, a germinating seed, a germinated seed, or a seedling of the transgenic plant.

2. A transgenic plant according to claim 1, wherein the hydrolytic enzyme hydrolyzes a substrate naturally occurring in the plant seed tissue of the transgenic plant.

3. A transgenic plant according to claim 1 or 2, wherein the plant is a rice, a barley, a rye, an oat, or a canola plant.

4. A transgenic plant according to any one of claims 1 to 3, wherein the promoter is an α-amylase gene promoter or a glutelin gene promoter.

5. A transgenic plant according to claim 4, wherein the α-amylase promoter is an *αAmy8* promoter.

6. A transgenic plant according to any one of the preceding claims, wherein the enzyme is a phytase, an amylopullulanase or an α-amylase.

7. A transgenic seed obtainable from a transgenic plant according to any one of the preceding claims.

8. A transgenic seed, the genomic DNA of which comprises a gene, the said gene comprising a promoter operably linked to a nucleotide sequence encoding a hydrolytic enzyme, wherein the promoter drives expression of the hydrolytic enzyme in the said transgenic seed.

9. A transgenic seed according to claim 7 or 8 which has been germinated.

10. A method for the preparation of a hydrolytic enzyme, which method comprises:
(i) providing a transgenic plant according to any one of claims 1 to 6;
(ii) harvesting a transgenic seed from the said transgenic plant; and
(iii) germinating the said transgenic seed to produce the hydrolytic enzyme.

11. A method according to claim 10, which further comprises isolating the hydrolytic enzyme from the said germinated transgenic seed.

12. A method for the preparation of a polypeptide, which method comprises:
(i) providing a transgenic plant, the genomic DNA of which comprises a gene, the said gene comprising an α-amylase or a glutelin promoter operably linked to a nucleotide sequence encoding a polypeptide;
(ii) harvesting a transgenic seed from the transgenic plant;
(iii) germinating the transgenic seed to produce a mature seed; and
(iv) isolating the polypeptide from the mature seed.

13. A method according to claim 12, wherein, in the isolating step (iv), the polypeptide is isolated from an embryo or endosperm of the mature seed.

14. A method for obtaining a first-generation transgenic plant, which method comprises:
(i) transferring into a plant cell a gene which comprises a promoter operably linked to a nucleotide sequence encoding a hydrolytic enzyme; and
(ii) regenerating the transgenic plant cell to give a transgenic plant, wherein the promoter drives expression of the hydrolytic enzyme in a developing seed, a germinating seed, a germinated seed, or a seedling of the transgenic plant.

15. A method for obtaining a transgenic progeny plant, which method comprises obtaining a second-generation transgenic progeny plant from a first-generation transgenic plant obtainable by a method according to claim 14, and optionally obtaining transgenic plants of one or more further generations from the second-generation progeny plant thus obtained.

16. A method according to claim 15, which method comprises:
(a) obtaining a transgenic seed from a first-generation transgenic plant obtainable by a method according to claim 14, thus obtaining a second-generation transgenic progeny plant from the transgenic seed; and/or
(b) propagating clonally a first-generation transgenic plant obtainable by a method according to claim 14 to give a second-generation progeny plant; and/or
(c) crossing a first-generation transgenic plant obtainable by a method according to claim 14 with another plant to give a second-generation progeny plant; and optionally
(d) obtaining transgenic progeny plants of one or more further generations from the second-generation progeny plant thus obtained.

17. A method for obtaining a transgenic plant seed, which method comprises obtaining a transgenic seed from a first-generation transgenic plant obtainable by a method according to claim 14 or from a transgenic progeny plant obtainable by a method according to claim 15 or 16.

18. A first-generation transgenic plant, a transgenic progeny plant or a transgenic plant seed obtainable by a method according to any one of claims 14 to 17.
